# EUROPÄISCHE PATENTSCHRIFT

(11) **EP 2 056 906 B1**
(45) Veröffentlichungstag und Bekanntmachung des Hinweises auf die Patenterteilung: **02.01.2019**
(21) Anmeldenummer: 07802028.6
(22) Anmeldetag: 31.08.2007
(51) Int. Cl.: A61M 5/42

(54) **VORRICHTUNG UND VERFAHREN ZUR ÜBERWACHUNG EINES GEFÄSSZUGANGS SOWIE VORRICHTUNG ZUR SCHAFFUNG EINES GEFÄSSZUGANGS**
DEVICE AND METHOD FOR MONITORING A VASCULAR ACCESS, AND DEVICE FOR CREATING A VASCULAR ACCESS
DISPOSITIF ET PROCEÉDÉ DE SURVEILLANCE DE L'ACCÈS À UN VAISSEAU ET DISPOSITIF DE FORMATION D'UN ACCÈS À UN VAISSEAU

(30) Priorität: 02.09.2006 DE 102006041265
(43) Veröffentlichungstag der Anmeldung: 13.05.2009
(73) Patentinhaber: Fresenius Medical Care Deutschland GmbH, 61352 Bad Homburg v.d.H. (DE)
(72) Erfinder: GRÖBER, Tobias, 63150 Heusenstamm (DE); KÖNIG, Christoph, 65207 Wiesbaden (DE)
(74) Vertreter: Oppermann, Frank
(86) Internationale Anmeldenummer: PCT/EP2007/007610
(87) Internationale Veröffentlichungsnummer: WO 2008/028594

(56) Entgegenhaltungen:
- EP-A- 1 736 185
- WO-A-01/68163
- WO-A-99/26686
- WO-A-99/29356
- WO-A-2007/107561
- FR-A- 2 622 457

## Beschreibung

Die Erfindung bezieht sich auf eine Vorrichtung zur Schaffung eines Gefäßzugangs für eine extrakorporale Blutbehandlung mit einer extrakorporalen Blutbehandlungsvorrichtung. Darüber hinaus betrifft die Erfindung eine Vorrichtung zur Überwachung eines Gefäßzugangs bei einer extrakorporalen Blutbehandlung sowie eine extrakorporale Blutbehandlungsvorrichtung mit einer derartigen Vorrichtung.

Auf dem Gebiet der Medizintechnik sind verschiedene extrakorporale Blutbehandlungsvorrichtungen bekannt, die über einen extrakorporalen Blutkreislauf verfügen. Zu den bekannten extrakorporalen Blutbehandlungsvorrichtungen zählen beispielsweise Dialysevorrichtungen und Zellseparatoren, die einen Zugang zu dem Gefäßsystem des Patienten erforderlich machen. Bei der extrakorporalen Blutbehandlung wird dem Patienten über eine arterielle Schlauchleitung mit einer arteriellen Punktionskanüle Blut entnommen, das dem Patienten über eine venöse Schlauchleitung mit einer venösen Punktionskanüle wieder zugeführt wird.

Trotz regelmäßiger Überwachung des Gefäßzuganges durch das Krankenhauspersonal besteht grundsätzlich die Gefahr, dass die venöse Punktionskanüle unbemerkt aus dem Blutgefäß des Patienten herausrutscht. Während ein Herausrutschen der arteriellen Kanüle mit einem Ansaugen von Luft in die arterielle Schlauchleitung verbunden ist, führt das Herausrutschen der venösen Kanüle zu dem gefürchteten Freifluss des Blutes in die Umgebung. Wenn das Herausrutschen der venösen Kanüle daher nicht sofort erkannt wird, besteht die Gefahr, dass der Patient verblutet.

Zur Überwachung des Gefäßzugangs sind verschiedene Vorrichtungen unterschiedlicher Ausbildung bekannt. Die bekannten Überwachungsvorrichtungen greifen im Allgemeinen auf die standardmäßig in den Blutbehandlungsvorrichtungen vorhandenen Sicherheitsvorrichtungen zurück, die bei einem nicht ordnungsgemäßen Gefäßzugang eine sofortige Unterbrechung des extrakorporalen Blutkreislaufs auslösen.

Aus der US 2004/0254513 A1 ist eine extrakorporale Blutbehandlungsvorrichtung mit einem extrakorporalen Blutkreislauf bekannt, die über eine Überwachungsvorrichtung für den arteriellen und venösen Gefäßzugang verfügt. Die bekannte Überwachungsvorrichtung weist zwei Elektroden auf, von denen die eine an der arteriellen und die andere an der venösen Schlauchleitung angeordnet ist, um über zwei Messleitungen eine elektrische Verbindung zwischen der Flüssigkeit in der jeweiligen Schlauchleitung und einer Überwachungseinheit herzustellen. Die Überwachungseinheit misst die Impedanz zwischen den beiden Elektroden, wobei auf einen nicht ordnungsgemäßen Gefäßzugang geschlossen wird, wenn die Impedanz nicht innerhalb vorgegebener Grenzen liegt.

Die US 2004/0254513 A1 beschreibt auch eine Anwendung in der Infusionstechnik mit nur einer Schlauchleitung. Die Druckschrift schlägt vor, an der Schlauchleitung stromauf der Punktionskanüle eine erste Elektrode zur Herstellung einer elektrischen Verbindung zu der in der Leitung fließenden Flüssigkeit und eine zweite Elektrode vorzusehen, die auf die Haut des Patienten aufgelegt wird. Die Überwachungseinheit ist an beide Elektroden angeschlossen und überwacht die Stärke des elektrischen Stroms, der von der ersten Elektrode an der Schlauchleitung durch das Blut innerhalb des Schlauchs, die Punktionskanüle und den Bereich des Körpers des Patienten zwischen der Punktionskanüle und der zweiten Elektrode fließt.

Die oben beschriebenen Vorrichtungen haben den Nachteil, dass sich die Schaffung der elektrisch leitenden Ankoppelstellen als relativ aufwendig erweist. So befasst sich die US 2004/0254513 A1 insbesondere mit der Verwendung leitfähiger Polymere zur Herstellung der elektrischen Verbindung zu dem Blut im Blutschlauch. Nachteilig ist auch, dass die bekannten Vorrichtungen das Anlegen separater Körperelektroden erforderlich machen. Die WO 2007/107561 A2, die einen nachveröffentlichten Stand der Technik nach Art. 54 (3) EPÜ darstellt, beschreibt eine Vorrichtung zur Injektion von Insulin, die einen Basiskörper aus elektrisch nicht leitendem Material aufweist, in den das distale Endstück einer Punktionskanüle eingesetzt ist. An dem Basiskörper ist ein elektrisch leitendes Kontaktelement vorgesehen, das elektrisch mit der Punktionskanüle verbunden ist. Die Injektionsvorrichtung zeichnet sich dadurch aus, dass beim Einstechen der Punktionskanüle ein geschlossener elektrischer Strompfad geschaffen wird, der die Punktionskanüle, den Basiskörper sowie den Körper des Patienten einschließt. Dabei umgreift der Patient das elektrisch leitende Kontaktelement an dem Basiskörper mit der Hand. Der Basiskörper ist aber nicht dazu bestimmt, auf die Haut des Patienten aufgelegt zu werden.

Aus der WO 99/29356 A ist eine Vorrichtung zur Überwachung eines Gefäßzugangs bekannt, die für die Überwachung der Flüssigkeitszufuhr drei elektrische Kontaktstellen vorsieht, von denen die erste Kontaktstelle an der zu dem Katheter führenden Flüssigkeitsleitung nahe des Katheters und die zweite Kontaktstelle am Ende der Flüssigkeitsleitung nahe des Reservoirs liegt und die dritte Kontaktstelle von einer Elektrode gebildet wird, die irgendwo am, z.B. als Hautoberflächenelektrode, oder im Körper angebracht ist. Die Überwachung des Gefäßzugangs beruht auf einer Messung des elektrischen Widerstands auf zwei Messstrecken, wobei die eine Messstrecke zwischen der ersten und zweiten Kontaktstelle und die andere Messstrecke zwischen der ersten und dritten Kontaktstelle liegen.

Die EP 1 736 185 A2, die ebenfalls einen nachveröffentlichten Stand der Technik darstellt, beschreibt eine Injektionsvorrichtung, die eine Injektionsnadel und einen Basiskörper mit zwei Flügelstücken (Punktionsflügel) aufweist. In den Punktionsflügel ist ein elektronisches Sensorsystem integriert, das eine komplexe Impedanzmessung oder eine optische Messung erlaubt, wenn die Punktionskanüle in den Gefäßzugange eingestochen oder aus dem Gefäßzugang herausgerutscht ist. Eine Impedanzmessung kann zwischen einem elektrischen Kontaktelement an dem Basiskörper und einem Referenzkontakt erfolgen, der mit Masse (ground potential) verbunden ist. Eine alternative Ausführungsform sieht eine Impedanzmessung zwischen einer metallischen Punktionskanüle und einem Kontakt in unmittelbarer Nähe zu der Kanüle vor.

Die WO 99/26686 beschreibt ein Pad zur Impedanzmessung, das auf die Haut des Patienten aufgelegt wird. Der Erfindung liegt die Aufgabe zu Grunde, einen Gefäßzugang ohne umfangreiche Veränderungen an der Blutbehandlungsvorrichtung sowie das Anlegen separater Körperelektroden mit hoher Zuverlässigkeit zu überwachen.

Die Lösung dieser Aufgabe erfolgt erfindungsgemäß mit den in den Patentansprüchen 1, 6 und 9 angegebenen Merkmalen. Bevorzugte Ausführungsformen sind Gegenstand der Unteransprüche.

Die Erfindung beruht darauf, dass die erforderlichen elektrischen Verbindungen allein durch die Vorrichtung zur Schaffung eines Gefäßzugangs bereitgestellt werden.

Ein erster Aspekt der Erfindung sieht eine Vorrichtung zur Schaffung eines Gefäßzugangs vor, die eine elektrisch leitende Punktionskanüle mit einem proximalen und distalen Endstück und einen Basiskörper aus elektrisch nicht leitendem Material aufweist, in den das distale Endstück der Punktionskanüle eingesetzt ist. Während die elektrisch leitende Punktionskanüle die erste Messelektrode bildet, weist der Basiskörper ein elektrisch leitendes Kontaktelement als zweite Messelektrode auf. Der Basiskörper der Punktionskanüle aus elektrisch nicht leitendem Material stellt einen Isolator zwischen der elektrisch leitenden Punktionskanüle und dem elektrischen Kontaktelement dar.

Ein ordnungsgemäßer Gefäßzugang setzt voraus, dass die elektrisch leitende Punktionskanüle sich innerhalb des Gefäßzugangs befindet, so dass Blut durch die Kanüle strömt, während der Basiskörper mit dem Kontaktelement auf dem Arm des Patienten aufliegt. Auf einen fehlerhaften Gefäßzugang wird dann geschlossen, wenn entweder die Punktionskanüle herausgerutscht ist (Diskonnektion) oder der Basiskörper nicht auf dem Arm des Patienten aufliegt (Dislokation). Wenn dies der Fall ist, ändert sich die Impedanz zwischen der Punktionskanüle und dem elektrisch leitenden Kontaktelement.

Die Vorrichtung zur Überwachung des Gefäßzugangs misst die Impedanz zwischen der Punktionskanüle und dem Kontaktelement und schließt bei einer Änderung der Impedanz innerhalb vorgegebener Grenzen auf einen ordnungsgemäßen Gefäßzugang, da ein nicht ordnungsgemäßer Gefäßzugang zu einer starken Änderung der Impedanz führt. Die gemessene Impedanz kann mit einem Grenzwert oder auch mehreren Grenzwerten verglichen werden. Vorzugsweise wird die Impedanz mit einem vorgegebenen unteren Grenzwert und einem vorgegebenen oberen Grenzwert verglichen, wobei bei Unterschreiten des unteren Grenzwertes oder Überschreiten des oberen Grenzwertes auf einen nicht ordnungsgemäßen Gefäßzugang geschlossen wird.

Da die Punktionskanüle im Allgemeinen aus Metall besteht, kann eine konventionelle Punktionskanüle Verwendung finden, die ohnehin leitfähig ist. Es ist aber auch möglich, die Punktionskanüle aus einem leitfähigen Polymer herzustellen.

Bei einer bevorzugten Ausführungsform weist der Basiskörper der Vorrichtung zur Schaffung des Gefäßzugangs ein zentrales Aufnahmestück auf, in den das distale Endstück der Punktionskanüle eingesetzt ist, und zwei Flügelstücke auf, die zu beiden Seiten von dem zentralen Aufnahmestück für die Punktionskanüle abstehen. Damit hat die bevorzugte Ausführungsform im Wesentlichen die Ausbildung der als Schmetterlingskanülen bekannten Vorrichtungen zur Schaffung eines Gefäßzugangs. Die beiden Flügelstücke (Punktionsflügel) sind im Allgemeinen flexibel ausgebildet und werden mit Daumen und Zeigefinger zum Anstechen des Gefäßes gehalten. Nach dem Anstechen des Gefäßes spreizen sich die Punktionsflügel wieder ab, so dass sie mit ihrer Unterseite flach auf der Haut des Patienten aufliegen.

Bei einer besonders bevorzugten Ausführungsform weisen die beiden Flügelstücke das Kontaktelement auf. Vorzugsweise ist das Kontaktelement an der auf den Patienten aufzulegenden Unterseite der beiden Flügelstücke vorgesehen, so dass eine elektrische Verbindung zwischen dem Kontaktelement und der Haut des Patienten hergestellt wird.

Das Kontaktelement kann aber auch mit einer nicht-leitenden Schicht isoliert sein. Grundsätzlich kann das Kontaktelement auch in die Flügelstücke eingebettet sein, beispielsweise in die aus Kunststoff bestehenden Flügelstücke eingegossen sein.

Das Kontaktelement kann unterschiedlich ausgebildet sein, beispielsweise kann das Kontaktelement eine elektrisch leitende Folie oder eine Beschichtung aus elektrisch leitendem Material sein. Das Kontaktelement kann auch aus mehreren Teilstücken bestehen, die elektrisch miteinander verbunden sind.

Um die Messleitungen mit der Überwachungseinheit der Überwachungsvorrichtung verbinden zu können, weisen Basiskörper und Flügelstücke vorzugsweise zwei Anschlussstücke auf, mit denen sich eine elektrische Verbindung zu der Punktionskanüle bzw. dem Kontaktelement herstellen lässt.

Wenn bei dem Anschluss der Überwachungseinheit der Überwachungsvorrichtung an die Vorrichtung zur Schaffung des Gefäßzugangs von zwei Messleitungen und zwei Anschlussstücken die Rede ist, ist darunter auch eine gemeinsame Anschlussleitung mit einem gemeinsamen Anschlussteil zu verstehen, das die beiden Anschlüsse umfasst.

Ein wesentlicher Vorteil liegt darin, dass die Verwendung der erfindungsgemäßen Vorrichtung zur Schaffung des Gefäßzugangs mit Ausnahme des Anschlusses der Messleitungen keinen zusätzlichen Aufwand erforderlich macht. Insbesondere ist es nicht erforderlich, Kontaktstellen an den Schlauchleitungen und separate Kontaktstellen am Patienten vorzusehen. Sämtliche Kontaktstellen sind bei der Erfindung integraler Bestandteil der Vorrichtung zur Schaffung des Gefäßzugangs, die vorzugsweise einen Einmalartikel (Disposable) bilden kann.

Im Allgemeinen wird die Vorrichtung zur Schaffung des Gefäßzugangs, beispielsweise in Form einer Schmetterlingskanüle, zusammen mit den Schlauchleitungen als Disposable zur Verfügung gestellt.

Die erforderlichen Komponenten für die Überwachungsvorrichtung, an die die Vorrichtung zur Schaffung des Gefäßzugangs über die Messleitungen angeschlossen wird, kann im Allgemeinen ebenfalls ohne großen Aufwand bereitgestellt werden, da die wesentlichen Komponenten bereits in den bekannten Blutbehandlungsvorrichtungen vorhanden sind.

Im Folgenden wird ein Ausführungsbeispiel der Erfindung unter Bezugnahme auf die Zeichnungen näher erläutert.

Es zeigen:
- Figur 1: eine Unteransicht einer vereinfachten schematischen Darstellung einer erfindungsgemäßen Vorrichtung zur Schaffung eines Gefäßzugangs,
- Figur 2: die Vorrichtung zur Schaffung eines Gefäßzugangs von Figur 1 in der Seitenansicht,
- Figur 3: eine Schaltungsanordnung zur Veranschaulichung des Funktionsprinzips der erfindungsgemäßen Vorrichtung zur Überwachung des Gefäßzugangs,
- Figur 4: das Ersatzschaltbild der Schaltungsanordnung von Figur 3,
- Figuren 5A bis 5C: die gemessenen Spannungen an dem Messwiderstand der Schaltungsanordnung von Figuren 3 und 4 für den Fall eines ordnungsgemäßen Patientenzugangs, einer Dislokation und einer Diskonnektion und
- Figur 6: die wesentlichen Komponenten einer erfindungsgemäßen extrakorporalen Blutbehandlungsvorrichtung mit einer erfindungsgemäßen Vorrichtung zur Überwachung eines Gefäßzugangs.

Die erfindungsgemäße Vorrichtung zur Schaffung eines Gefäßzugangs, die bei dem beschriebenen Ausführungsbeispiel in Form einer sog. Schmetterlingskanüle ausgebildet ist, weist eine elektrisch leitende Punktionskanüle 1 aus Metall mit einem angespitzten proximalen Endstück 1A und einem distalen Endstück 1B sowie einen Basiskörper 2 auf, der ein Kunststoff-Spritzgießteil ist, in das das distale Endstück 1B eingesetzt ist.

Der Basiskörper 2 weist ein rohrförmiges, zentrales Aufnahmestück 3 auf, in dem das distale Endstück 1B der Punktionskanüle 1 sitzt. An das zentrale Aufnahmestück 3 für die Punktionskanüle 1 sind zu beiden Seiten jeweils ein flaches Flügelstück 4, 5 angeformt.

Die beiden Flügelstücke 4, 5 weisen eine flache Unterseite 6 auf, mit der der Basiskörper 2 auf die Haut des Patienten aufgelegt wird. Auf die Unterseite 6 der Flügelstücke 4, 5 des Basiskörpers 3 ist ein elektrisch leitendes Kontaktelement 7 aufgebracht, das beispielsweise eine Schicht ist, die von einem elektrisch leitenden Material gebildet wird. Das Kontaktelement 7 erstreckt sich im Wesentlichen über die gesamte Unterseite der Flügelstücke 4, 5 sowie des Basiskörpers 2.

Darüber hinaus verfügt die Schmetterlingskanüle über ein erstes Anschlussstück 8, das mit dem Abschnitt des über den Basiskörper 2 hinaus vorstehenden distalen Endstücks 1B der Punktionskanüle 1 elektrisch verbunden ist, während ein zweites Anschlussstück 9 an einem der beiden Punktionsflügel 4, 5 mit dem Kontaktelement 7 elektrisch verbunden ist. Beide Kontaktstücke 8, 9, die nur schematisch dargestellt sind, dienen zum Anschluss jeweils einer nicht dargestellten Messleitung zum Verbinden der Schmetterlingskanüle mit der Überwachungseinheit der Vorrichtung zur Überwachung des Gefäßzugangs, die noch im Einzelnen beschrieben wird.

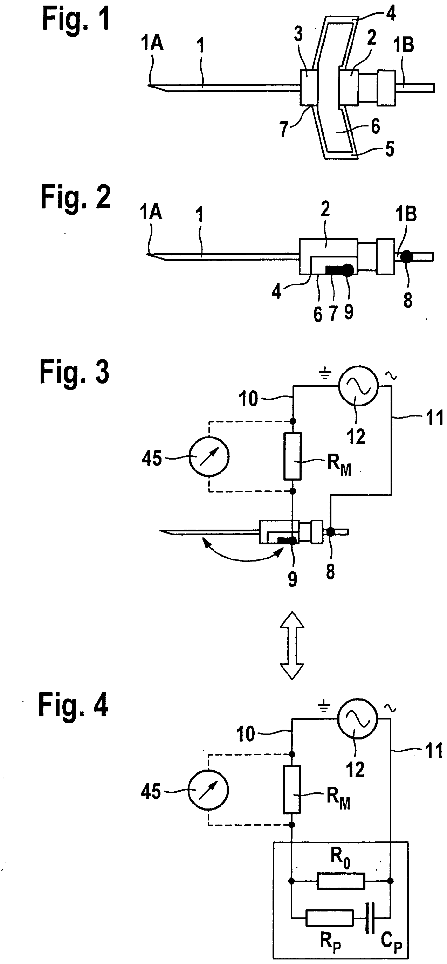

Nachfolgend wird die Funktionsweise der erfindungsgemäßen Überwachungsvorrichtung unter Bezugnahme auf die Figuren 3 und 4 sowie die Figuren 5A bis 5C im Einzelnen erläutert.

Das Messprinzip beruht auf einer Impedanzmessung des Patienten zwischen der Punktionskanüle 1 und dem Kontaktelement 7, das auf der Haut des Patienten an dessen Oberarm aufliegt.

Figur 3 zeigt eine Schaltungsanordnung zur Veranschaulichung des Messprinzips. Die beiden Anschlüsse 8, 9 der Schmetterlingskanüle sind über Messleitungen 10, 11 mit einem Frequenzgenerator 12 verbunden, der ein Wechselspannungssignal liefert. In eine der beiden Messleitungen ist ein Messwiderstand R_{M} geschaltet.

Mit einem Oszilloskop 13 wird die an den Messwiderstand R_{M} abfallende Spannung gemessen. Dabei wird davon ausgegangen, dass zwischen den beiden Anschlussstücken 8, 9 eine elektrische Verbindung durch den Patienten hergestellt wird. Im Falle einer Diskonnektion oder Dislokation der Punktionskanüle ändert sich folglich die Impedanz, was durch eine Abnahme der Amplitude des mit dem Oszilloskop gemessenen Signals nachgewiesen wird. So wird bei einer Diskonnektion der Nadel die Impedanz größer.

Figur 4 zeigt ein Ersatzschaltbild, der über den Patienten zwischen den Anschlussstücken 8, 9 der Schmetterlingskanüle hergestellten leitenden Verbindung. Das Ersatzschaltbild des "Patienten" ist die Parallelschaltung einer Serienschaltung eines Widerstandes Rp und eines Kondensators C_{P} einerseits und eines Widerstandes R₀ andererseits.

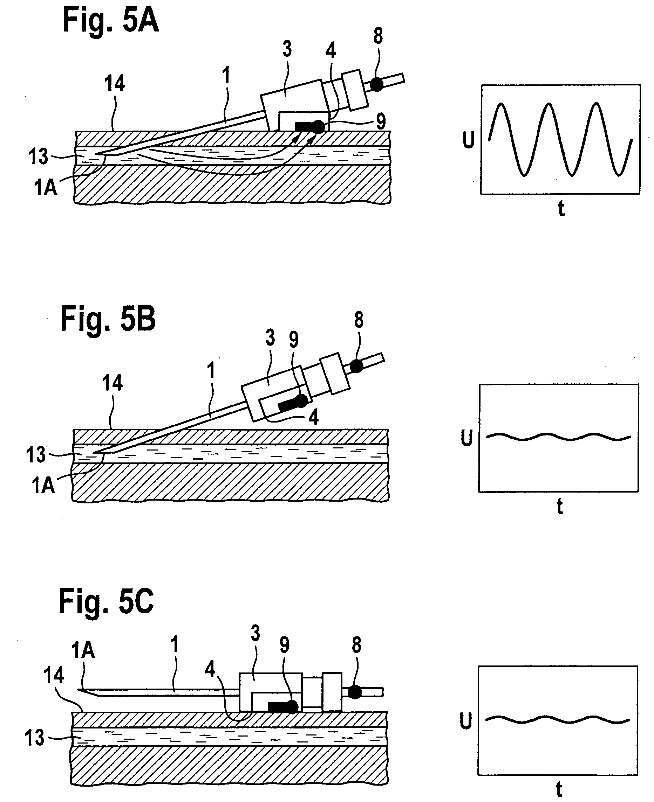

Die Figuren 5A bis 5C zeigen die Lage der Schmetterlingskanüle und das mit dem Oszilloskop gemessene Spannungssignal.

Wenn der Patientenzugang ordnungsgemäß ist (Figur 5A), d.h. die Punktionskanüle 1 befindet sich in dem Gefäß 13 des Patienten und das Kontaktelement 7 liegt auf der Haut 14 des Patienten auf, wird ein Wechselspannungssignal mit einer relativ großen Amplitude gemessen. Das gemessene Wechselspannungssignal ist in Figur 5A in der rechten Bildhälfte dargestellt.

Im Falle einer Dislokation der Schmetterlingskanüle, d.h. die Punktionskanüle 1 befindet sich zwar noch im Gefäß 13, der Basiskörper 2 mit dem Kontaktelement 2 liegt aber nicht mit dem Kontaktelement 7 mehr auf der Haut 14 des Patienten auf (Figur 5B), wird ein Wechselspannungssignal mit einer geringeren Amplitude gemessen. Das gemessene Wechselspannungssignal ist wieder in Figur 5B in der rechten Bildhälfte dargestellt.

Für den Fall einer Diskonnektion, d.h. die Punktionskanüle 1 ist aus dem Gefäß 13 herausgerutscht, wird das in Figur 5C auf der rechten Bildhälfte dargestellte Signal gemessen. Das Signal weist wieder eine wesentlich geringere Amplitude als das Signal auf, das bei einem ordnungsgemäßen Gefäßzugang gemessen wird (Figur 5A). Die Amplitude des gemessenen Signals ist selbst dann wesentlich geringer, wenn das Kontaktelement 7 des Basiskörpers 2 noch auf der Haut 14 des Patienten aufliegen sollte.

Folglich kann durch Vergleich der Amplitude des gemessenen Wechselspannungssignals mit vorgegebenen Grenzwerten zuverlässig erkannt werden, ob der Patientenzugang ordnungsgemäß ist oder nicht.

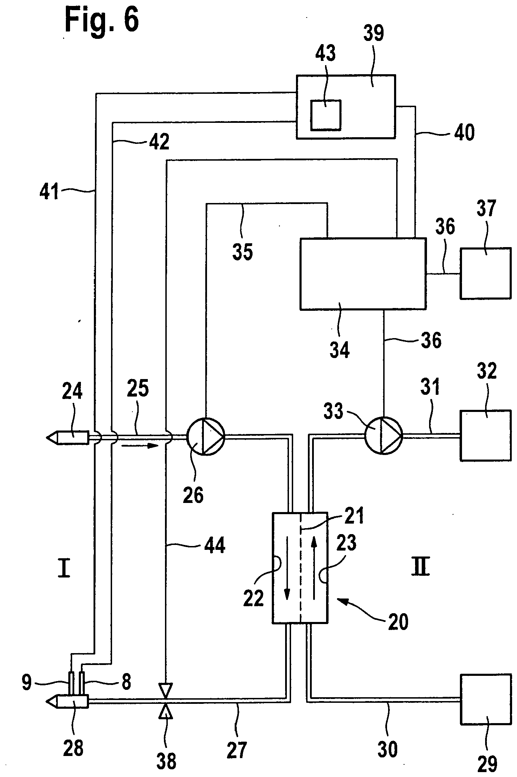

Nachfolgend wird die erfindungsgemäße extrakorporale Blutbehandlungsvorrichtung beschrieben, die über eine Vorrichtung zur Überwachung des Gefäßzugangs verfügt. Figur 6 zeigt nur die wesentlichen Komponenten der Blutbehandlungsvorrichtung in schematischer Darstellung, da Blutbehandlungsvorrichtungen als solche dem Fachmann bekannt sind. Die unter Bezugnahme auf die Figuren 1 und 2 beschriebene Vorrichtung zur Herstellung eines Gefäßzugangs ist in Figur 6 ebenfalls nur schematisch dargestellt.

Bei der Blutbehandlungsvorrichtung handelt es sich um eine bekannte Hämodialysevorrichtung, die einen Dialysator 20 aufweist, der durch eine semi-permeable Membran 21, in eine Blutkammer 22 und eine Dialysierflüssigkeitskammer 23 unterteilt ist.

Das Blut des Patienten wird mittels einer ersten Vorrichtung zur Schaffung eines Gefäßzugangs 24 (Schmetterlingskanüle) entnommen, das über eine an die Schmetterlingskanüle 24 angeschlossene arterielle Blutleitung 25, in die eine Blutpumpe 26 geschaltet ist, dem Einlass der Blutkammer 22 des Dialysators 20 zugeführt wird. Von dem Auslass der Blutkammer 22 des Dialysators 20 geht eine venöse Blutleitung 27 ab, die zu der venösen Schmetterlingskanüle 28 führt. Die Blutpumpe 26 fördert das Blut des Patienten im extrakorporalen Kreislauf I der Dialysevorrichtung.

Der Dialysierflüssigkeitskreislauf II der Dialysevorrichtung umfasst eine Dialysierflüssigkeitsquelle 29, an der eine Dialysierflüssigkeitszuführleitung 30 angeschlossen ist, die zu dem Einlass der Dialysierflüssigkeitskammer 23 des Dialysators 20 führt. Von dem Auslass der Dialysierflüssigkeitskammer 23 des Dialysators geht eine Dialysierflüssigkeitsabführleitung 31 ab, die zu einem Auslass 32 führt. In die Dialysierflüssigkeitsabführleitung 31 ist eine Dialysierflüssigkeitspumpe 33 geschaltet.

Die Steuerung der Dialysevorrichtung übernimmt eine zentrale Steuereinheit 34, die über Steuerleitungen 35, 36 die Blut- und Dialysierflüssigkeitspumpe 26, 33 ansteuert. Die zentrale Steuereinheit 34 ist über eine Datenleitung 36 mit einer Alarmeinheit 37 verbunden, die bei einem Störfall einen optischen und/oder akustischen Alarm gibt.

Stromab der Blutkammer 22 des Dialysators 20 befindet sich an der venösen Schlauchleitung 27 eine elektromagnetisch betätigbare Schlauchklemme 38, die über eine weitere Steuerleitung 39 von der zentralen Steuereinheit 34 geschlossen wird, wenn die venöse Punktionskanüle 28 aus dem Gefäßzugang herausrutschen sollte. Darüber hinaus stoppt die Steuereinheit 34 nach dem Herausrutschen der Kanüle die Blutpumpe 26.

Die oben beschriebenen Komponenten sind im Allgemeinen in den bekannten Dialysevorrichtungen bereits vorhanden.

Die erfindungsgemäße Vorrichtung zur Überwachung des Gefäßzugangs umfasst neben der erfindungsgemäßen Vorrichtung zur Schaffung des venösen Gefäßzugangs 28 (Schmetterlingskanüle) eine Überwachungseinheit 39, die über eine weitere Datenleitung 40 mit der zentralen Steuereinheit 34 der Dialysemaschine kommuniziert. Die Überwachungseinheit 39 ist über zwei Messleitungen 41, 42 mit Schmetterlingskanüle 28 verbunden, wobei die beiden Messleitungen 41, 42 an deren Anschlussstücken 8, 9 angeschlossen sind. Die Überwachungseinheit 39 führt eine unter Bezugnahme auf die Figuren 3 und 4 beschriebene Impedanzmessung durch, wobei die Amplitude des an einem Messwiderstand abfallenden Spannungssignals gemessen wird. Die Überwachungseinheit verfügt über eine Vergleichseinrichtung 43, mit der das gemessene Spannungssignal mit einem vorgegebenen Grenzwert verglichen wird. Für den Fall, dass der vorgegebene Grenzwert unterschritten wird, erzeugt die Überwachungseinheit 39 ein Alarmsignal, das über die Datenleitung 40 an die zentrale Steuereinheit 34 übertragen wird. Die zentrale Steuereinheit 34 steuert daraufhin die Alarmeinheit 37 an, die einen optischen und/oder akustischen Alarm gibt. Darüber hinaus steuert die Steuereinheit 34 die venöse Schlauchklemme 38 an, die die venöse Schlauchleitung 27 unterbricht.

Bei einer alternativen Ausführungsform wird die gemessene Impedanz sowohl mit einem unteren Grenzwert als auch mit einem oberen Grenzwert verglichen, die einen Toleranzbereich definieren. Wenn die Impedanz innerhalb des Toleranzbereiches liegt, d.h. die Impedanz kleiner als der oberen Grenzwert und größer als der untere Grenzwert ist, wird ein ordnungemäßer Gefäßzugang angenommen. Verlässt die Impedanz hingegen den Toleranzbereich, wird von einem nicht ordnungsgemäßen Gefäßzugang ausgegangen.

Die erfindungsgemäße Überwachungsvorrichtung, die von der erfindungsgemäßen Vorrichtung zur Schaffung eines Gefäßzugangs (Schmetterlingskanüle) Gebrauch macht, zeichnet sich dadurch aus, dass selbst dann eine Diskonnektion oder Dislokation erkannt werden kann, wenn das Schlauchsystem nicht mit Blut befüllt ist, sondern sich Luft in der venösen Schlauchleitung befindet.

## Patentansprüche

1. Vorrichtung zur Schaffung eines Gefäßzugangs für eine extrakorporale Blutbehandlung mit einer extrakorporalen Blutbehandlungsvorrichtung, wobei die Vorrichtung zur Schaffung des Gefäßzugangs eine elektrisch leitende Punktionskanüle (1) mit einem proximalen und distalen Endstück (1A, 1B) und
einen Basiskörper (2) aus elektrisch nicht leitendem Material aufweist, in den das distale Endstück der Punktionskanüle eingesetzt ist, wobei der Basiskörper (2) ein elektrisch leitendes Kontaktelement (7) aufweist,
**dadurch gekennzeichnet, dass**
die elektrisch leitende Punktionskanüle (1) eine erste Messelektrode bildet und das elektrisch leitende Kontaktelement (7) des Basiskörpers (2) eine zweite Messelektrode bildet, wobei der Basiskörper (2) aus elektrisch nicht leitendem Material einen Isolator zwischen der elektrisch leitenden Punktionskanüle (1) und dem elektrischen Kontaktelement bildet.

2. Vorrichtung nach Anspruch 1, **dadurch gekennzeichnet, dass** der Basiskörper (2) ein zentrales Aufnahmestück (3), in den das distale Endstück (1B) der Punktionskanüle (1) eingesetzt ist, und zwei Flügelstücke (4, 5) aufweist, die zu beiden Seiten von dem zentralen Aufnahmestück abstehen.

3. Vorrichtung nach Anspruch 2, **dadurch gekennzeichnet, dass** die beiden Flügelstücke (4, 5) das Kontaktelement (7) aufweisen.

4. Vorrichtung nach Anspruch 3, **dadurch gekennzeichnet, dass** das Kontaktelement (7) an der auf den Patienten aufzulegenden Unterseite der beiden Flügelstücke (4, 5) vorgesehen ist.

5. Vorrichtung nach einem der Ansprüche 1 bis 4, **dadurch gekennzeichnet, dass** der Basiskörper ein erstes Anschlussstück (8) zum Anschluss einer ersten Messleitung an die Punktionskanüle (1) und ein zweites Anschlussstück (9) zum Anschluss einer zweiten Messleitung an das Kontaktelement (7) aufweist.

6. Vorrichtung zur Überwachung eines Gefäßzugangs bei einer extrakorporalen Blutbehandlung mit einer Vorrichtung zur Schaffung eines Gefäßzugangs nach einem der Ansprüche 1 bis 5 und einer mit einer ersten und zweiten Messleitung (41, 42) an die Vorrichtung zur Schaffung des Gefäßzugangs angeschlossenen Überwachungseinheit (39) zum Messen der Impedanz zwischen der Punktionskanüle (1) und dem Kontaktelement (7), wobei die Überwachungseinheit bei einer Änderung der Impedanz innerhalb vorgegebener Grenzen auf das Vorliegen eines ordnungsgemäßen Gefäßzugangs schließt.

7. Vorrichtung zur Überwachung eines Gefäßzugangs nach Anspruch 6, **dadurch gekennzeichnet, dass** die Überwachungseinheit (39) eine Vergleichseinrichtung (43) aufweist, mit der die gemessene Impedanz mit einem vorgegebenen Grenzwert verglichen wird, wobei bei Unterschreiten des Grenzwertes auf einen nicht ordnungsgemäßen Gefäßzugang geschlossen wird.

8. Vorrichtung zur Überwachung eines Gefäßzugangs nach Anspruch 6 oder 7, **dadurch gekennzeichnet, dass** die Überwachungseinheit (39) eine Vergleichseinrichtung (43) aufweist, mit der die gemessene Impedanz mit einem vorgegebenen unteren Grenzwert und einem vorgegebenen oberen Grenzwert verglichen wird, wobei bei Unterschreiten des unteren Grenzwertes oder Überschreiten des oberen Grenzwertes auf einen nicht ordnungsgemäßen Gefäßzugang geschlossen wird.

9. Extrakorporale Blutbehandlungsvorrichtung mit einer Vorrichtung zur Überwachung eines Gefäßzugangs nach einem der Ansprüche 6 bis 8.

## Claims

1. Device for creating a vessel access for extracorporeal blood treatment with an extracorporeal blood treatment device, wherein the device for creating the vessel access has an electrically conductive puncture needle (1) with a proximal and distal end piece (1A, 1B) and a base body (2) made of electrically non-conductive material, into which the distal end piece of the puncture needle is inserted, the base body (2) having an electrically conductive contact element (7),
**characterised in that**
the electrically conductive puncture needle (1) forms a first measurement electrode and the electrically conductive contact element (7) of the base body (2) forms a second measurement electrode, the base body (2) made of electrically non-conductive material represents an insulator between the electrically conductive puncture needle (1) and the electrical contact element.

2. Device according to claim 1, **characterised in that** the base body (2) has a central retainer piece (3) into which the distal end piece (1B) of the puncture needle (1) is inserted, and two wing pieces (4, 5) that project away at both sides from the central retainer piece.

3. Device according to claim 2, **characterised in that** the two wing pieces (4, 5) have the contact element (7).

4. Device according to claim 3, **characterised in that** the contact element (7) is provided on the underside of the two wing pieces (4, 5), which is to be placed on the patient.

5. Device according to one of the claims 1 to 4, **characterised in that** the base body has a first connecting piece (8) for connecting a first measurement line to the puncture needle (1) and a second connecting piece (9) for connecting a second measurement line to the contact element (7).

6. Device for monitoring a vessel access in the case of extracorporeal blood treatment with a device according to one of the claims 1 to 5 for creating a vessel access, and with a monitoring unit (39) which is connected by a first and second measurement line (41, 42) to the device for creating the vessel access and which is for measuring the impedance between the puncture needle (1) and the contact element (7), wherein the monitoring unit deduces an incorrect vessel access in the event of a change in impedance within specified limits.

7. Device for monitoring a vessel access according to claim 6, **characterised in that** the monitoring unit (39) has a comparison unit (43) with which the measured impedance is compared to a specified threshold value, wherein incorrect vessel access is deduced if the value falls below the threshold value.

8. Device for monitoring a vessel access according to claim 6 or 7, **characterised in that** the monitoring unit (39) has a comparison unit (43) with which the measured impedance is compared to a specified lower threshold value and a specified upper threshold value, wherein incorrect vessel access is deduced if the measured value falls below the lower threshold value or exceeds the upper threshold value.

9. Extracorporeal blood treatment device with a device for monitoring a vessel access according to one of the claims 6 to 8.

## Revendications

1. Dispositif de formation d'un accès à un vaisseau pour un traitement sanguin extracorporel avec un dispositif de traitement sanguin extracorporel, dans lequel le dispositif de formation de l'accès à un vaisseau présente une canule de ponction (1) conductrice électriquement avec un embout proximal et distal (1A, 1B) et un corps de base (2) en matériau non conducteur électriquement, dans lequel l'embout distal de la canule de ponction est inséré, le corps de base (2) présentant un élément de contact (7) conducteur électriquement,
**caractérisé en ce que**
la canule de ponction (1) conductrice électriquement forme une première électrode de mesure et l'élément de contact (7) conducteur électriquement du corps de base (2) forme une deuxième électrode de mesure, dans lequel le corps de base (2) en matériau non conducteur électriquement forme un isolant entre la canule de ponction (1) conductrice électriquement et l'élément de contact électrique.

2. Dispositif selon la revendication 1, **caractérisé en ce que** le corps de base (2) présente une pièce de réception centrale (3), dans laquelle l'embout distal (1B) de la canule de ponction (1) est inséré, et deux pièces à ailette (4, 5), qui s'étendent des deux côtés à partir de la pièce de réception centrale.

3. Dispositif selon la revendication 2, **caractérisé en ce que** les deux pièces à ailette (4, 5) présentent l'élément de contact (7).

4. Dispositif selon la revendication 3, **caractérisé en ce que** l'élément de contact (7) est prévu au niveau de la face inférieure à poser sur le patient des deux pièces à ailette (4, 5).

5. Dispositif selon l'une quelconque des revendications 1 à 4, **caractérisé en ce que** le corps de base présente un premier raccord (8) pour raccorder une première ligne de mesure à la canule de ponction (1) et un deuxième raccord (9) pour raccorder une deuxième ligne de mesure à l'élément de contact (7).

6. Dispositif de surveillance d'un accès à un vaisseau lors d'un traitement sanguin extracorporel avec un dispositif de formation d'un accès à un vaisseau selon l'une quelconque des revendications 1 à 5 et une unité de surveillance (39) raccordée à une première et deuxième ligne de mesure (41, 42) au niveau du dispositif de formation de l'accès à un vaisseau pour mesurer l'impédance entre la canule de ponction (1) et l'élément de contact (7), dans lequel l'unité de surveillance conclut à la présence d'un accès à un vaisseau correct en cas de variation de l'impédance à l'intérieur de limites prédéfinies.

7. Dispositif de surveillance de l'accès à un vaisseau selon la revendication 6, **caractérisé en ce que** l'unité de surveillance (39) présente un dispositif de comparaison (43), avec lequel l'impédance mesurée est comparée à une valeur limite prédéfinie, dans lequel il est conclu à un accès à un vaisseau non correct en cas de sous-dépassement de la valeur limite.

8. Dispositif de surveillance de l'accès à un vaisseau selon la revendication 6 ou 7, **caractérisé en ce que** l'unité de surveillance (39) présente un dispositif de comparaison (43), avec lequel l'impédance mesurée est comparée à une valeur limite inférieure prédéfinie et une valeur limite supérieure prédéfinie, dans lequel il est conclu à un accès à un vaisseau non correct en cas de sous-dépassement de la valeur limite inférieure ou de dépassement de la valeur limite supérieure.

9. Dispositif de traitement sanguin extracorporel avec un dispositif de surveillance de l'accès à un vaisseau selon l'une quelconque des revendications 6 à 8.
